# EUROPÄISCHE PATENTSCHRIFT

(11) **EP 0 501 265 B1**
(45) Veröffentlichungstag und Bekanntmachung des Hinweises auf die Patenterteilung: **02.11.1994**
(21) Anmeldenummer: 92102585.4
(22) Anmeldetag: 17.02.1992
(51) Int. Cl.: B01J 23/64, C07C 209/72, C07C 209/26

(54) **Verfahren zur Herstellung eines Gemisches aus Cyclohexylamin und Dicyclohexylamin unter Einsatz eines Edelmetall-Trägerkatalysators**
Method for the production of a mixture of cyclohexyl amine and dicyclohexyl amine using a noble metal supported catalyst
Procédé de production d'un mélange de cyclohexylamine et dicyclohhexylamine en utilisant un catalyseur supporté à base de métaux nobles

(30) Priorität: 01.03.1991 DE 4106543
(43) Veröffentlichungstag der Anmeldung: 02.09.1992
(73) Patentinhaber: BAYER AG, 51368 Leverkusen (DE)
(72) Erfinder: Immel, Otto, Dr., W-4150 Krefeld (DE); Darsow, Gerhard, Dr., W-4150 Krefeld (DE); Waldmann, Helmut, Dr., W-5090 Leverkusen 1 (DE); Petruck, Gerd-Michael, Dr., W-4006 Erkrath 2 (DE)

(56) Entgegenhaltungen:
- EP-A- 0 227 868
- EP-A- 0 324 984
- FR-A- 1 333 693
- FR-A- 2 115 860
- US-A- 4 609 679

## Beschreibung

Die Erfindung betrifft die Verwendung eines Trägerkatalysators, der Ruthenium, Palladium oder ein Gemisch beider Metalle enthält und einen Träger aus Niobsäure, Tantalsäure oder einem Gemisch beider hat, in einem Verfahren zur Herstellung eines Gemisches aus gegebenenfalls substituiertem Cyclohexylamin und gegebenenfalls substituiertem Dicyclohexylamin durch katalytische Hydrierung von gegebenenfalls substituiertem Anilin.

Es ist bekannt, Cyclohexylamin und andere kernhydrierte Aminoverbindungen durch katalytische Hydrierung von Anilin und anderen aromatischen Aminoverbindungen herzustellen. Als Katalysatoren sind hierfür bekannt: Kobalt-Katalysatoren, die einen basischen Zusatzstoff enthalten (GB 969 542), Raney-Kobalt (JP 68/03180), Ruthenium-Katalysatoren (DE-AS 1 106 319), mit Alkalimetallverbindungen dotierte Ruthenium-Katalysatoren (US 3.636.108) oder Nickel-Katalysatoren (DE-PS 805 518).

Die meisten der genannten Verfahren werden unter Druck betrieben und ergeben hauptsächlich Cyclohexylamin neben nur wenig Dicyclohexylamin. Das Dicyclohexylamin wird daher vielfach durch andere Verfahren hergestellt, so beispielsweise durch Druckhydrierung von Diphenylamin unter Verwendung eines Ruthenium-Katalysators (DE-AS 1 106 319). Weiterhin entsteht Dicyclohexylamin bei der Umsetzung von Cyclohexanon mit Cyclohexylamin in Gegenwart eines Palladium/Kohle-Katalysators unter einem Wasserstoffdruck von etwa 4 bar (FR 1.333.692). Das Verfahren der genannten DE-PS 805 518 ist hauptsächlich auf die Gewinnung von Dicyclohexylamin ausgerichtet, arbeitet jedoch mit umständlichen Nebenproduktrückführungen.

Aus EP 324 984 ist ein weiterer Katalysator für die Anilinhydrierung zu Cyclohexylamin und Dicyclohexylamin bekannt, der Ruthenium und Palladium sowie basische Alkalimetallverbindung auf Trägern, wie Al₂O₃, Aluminiumspinell, Aktivkohle und anderen, enthält.

Weitere Nachteile der genannten Verfahren bestehen in zum Teil beträchtlichen Mengen an Cyclohexan-Abfallprodukt, sowie in der unbefriedigenden Standzeit der eingesetzten Katalysatoren. Es bestand daher der Wunsch, ein in technischem Maßstab brauchbares Verfahren zu entwickeln, bei welchem der Verlust durch die Bildung von Cyclohexan zurückgedrängt wird und die Standzeit des verwendeten Katalysators verbessert ist, sowie ein Verfahren zu entwickeln, bei dem gemeinsam Cyclohexylamin und Dicyclohexylamin in Mengen gebildet werden, die je nach dem Bedarf der beiden genannten Stoffe variabel sind.

Überraschenderweise wurde gefunden, daß die genannten Anforderungen durch den Einsatz des im folgenden beschriebenen Edelmetall-Trägerkatalysators erfüllt werden.

Die Erfindung betrifft demnach ein Verfahren zur Herstellung eines Gemisches aus gegebenenfalls substituiertem Cyclohexylamin und gegebenenfalls substituiertem Dicyclohexylamin durch katalytische Hydrierung von gegebenenfalls substituiertem Anilin an einem Edelmetall-Trägerkatalysator, das dadurch gekennzeichnet ist, daß man einen Ruthenium, Palladium oder ein Gemisch beider enthaltenden Katalysator auf einem Träger, der Niobsäure Nb₂O₅.nH₂O, Tantalsäure Ta₂O₅.nH₂O oder ein Gemisch beider enthält, der das(die) Edelmetall(e) in einer Gesamtmenge von 0,05-5 Gew.-%, bevorzugt 0,1-4 Gew.-%, besonders bevorzugt 0,1-3 Gew.-% und für den Fall eines Ru/Pd-Gemisches beide Metalle in einem Gewichtsverhältnis von 1:9 - 9:1, bevorzugt 2:8 - 8:2, besonders bevorzugt 3:7 - 7:3 enthält, wobei alle Prozentsätze auf das Gesamtgewicht des Katalysators bezogen sind, einsetzt und bei einer Temperatur im Bereich von 100-340°C, bevorzugt 130-240°C und bei einem Druck von 0,5-500 bar, bevorzugt 2-400 bar, besonders bevorzugt 100-400 bar, ganz besonders bevorzugt 150-350 bar, arbeitet.

Die erfindungsgemäß einsetzbaren Katalysatoren sind demnach vor allem durch die Kombination von Pd und/oder Ru mit Niobsäure und/oder Tantalsäure ausgezeichnet. Solche Katalysatoren liefern gegenüber den bekannten Trägerkatalysatoren höhere Ausbeuten an Dicyclohexylamin.

Es ist insbesondere ein Merkmal des erfindungsgemäß eingesetzlen Katalysators, Niobsäure oder Tantalsäure als Katalysatorträger oder als Inhaltsstoff(e) solcher Träger einzusetzen. Bekanntlich ist Niobsäure ein Niobpentoxidhydrat (Nb₂O₅.nH₂O), das beispielsweise durch Behandlung wäßriger Lösungen von Niobsäuresalzen mit starken Mineralsäuren oder durch Behandlung von Niobalkoholaten, Niobsäurehalogeniden oder Niobsäureestern mit Wasser, Säuren oder Basen erhalten werden kann. So gefällte Niobsäure wird getrocknet und stellt sodann eine schwerlösliche feste Verbindung dar, deren restlicher Wassergehalt nicht definiert ist, obwohl eine so hergestellte Niobsäure äußerlich ein trockenes Pulver darstellt. Die Herstellung der Niobsäure (Niobpentoxidhydrat) wird beispielsweise in Gmelins Handbuch der anorg. Chemie, 8. Auflage, Niob, Teil B1, S. 49, beschrieben.

Tantalpentoxidhydrat (Tantalsäure) für die Herstellung des eingesetzlen Katalysators kann durch Hydrolyse von Tantal(V)-Salzen, Tantal(V)-Alkoholaten oder anderen geeigneten hydrolysierbaren Tantal(V)-Verbindungen hergestellt werden. Die Hydrolyse kann in analoger Weise wie bei den Niobverbindungen vorgenommen werden. Die Durchführung einer solchen Hydrolyse ist dem Fachmann wie für Nb grundsätzlich bekannt und beispielsweise in Gmelins-Handbuch der anorg. Chemie, 8. Auflage, Tantal, Teil B1, (1970), S. 53 und Chem. Lett. 1988, S. 1573, beschrieben. Das für eines der beiden Elemente Beschriebene gilt grundsätzlich auch für das jeweils andere. Die große chemische Ähnlichkeit beider Elemente und ihrer Verbindungen drückt sich auch darin aus, daß sie in ihren natürlichen Vorkommen weitgehend miteinander vergesellschaftet sind.

In bevorzugter Weise wird als Träger oder als Inhaltsstoff eines Trägers Niobsäure eingesetzt, insbesondere solche, die einen aus der natürlichen Herkunft stammenden Anteil an Tantalsäure von 0,0001 - 10 Mol-%, bezogen auf die Summe der Molzahl Niob- und Tantalsäure, enthält.

Damit die Niob- oder Tantalsäure in die für den Einsatz als Festbett-Katalysator günstige stückige Form gebracht werden kann, wird beispielsweise der feuchte Niederschlag der Hydrolyse in einem Kneter durchgeknetet und in einem Granulierapparat zu Formlingen verarbeitet. Die feuchten Formkörper werden anschließend beispielsweise bei 120°C getrocknet und 0,5 - 5 Stunden bei 200-400°C kalziniert. Hierbei entsteht eine BET-Oberfläche von 5 - 350 cm²/g. Zur Herstellung von Granulaten, Extrudaten oder Kugeln kann Niob- oder Tantalsäure auch mit einem Bindemittel verpreßt und granuliert werden.

Niob- bzw. Tantalsäure ist bezüglich des erfindungsgemäß einsetzbaren Katalysators eine aktive Substanz, deren Aktivität auch durch Vermischen mit anderen Feststoffen erhalten bleibt. Geeignete Feststoffe sind beispielsweise Titandioxid, Zinkoxid, Magnesiumoxid, Eisenoxid, Siliziumdioxid, Graphit, Al₂O₃ und andere. Diese Feststoffe können auch in der oben beschriebenen Weise als Bindemittel eingesetzt werden, Gemische der Niob- bzw. Tantalsäure mit diesen inerten Feststoffen können im Verhältnis von 5:95 - 99:1, bevorzugt 50:50 - 98:2, eingesetzt werden. Damit stehen erfindungsgemäß auch Träger für die beschriebenen Katalysatoren zur Verfügung, in denen Niob- bzw. Tantalsäure oder ein Gemisch beider nur einen Teil des Trägers darstellen. Als solche Träger seien besonders Al₂O₃/Niob- bzw. Tantalsäure-Träger erwähnt, die etwa durch Vermischen und Verpressen der Komponenten solcher Träger oder durch Auftränken und Ausfällen von Niob- bzw. Tantalverbindungen als Niob- bzw. Tantalsäure auf Al₂O₃ hergestellt werden können. Als Al₂O₃ wird das γ-Al₂O₃ bevorzugt. Der Gehalt an Niob- bzw. Tantalsäure ist in weiten Grenzen variierbar, beträgt aber bevorzugt 1-10 Gew.-%, bezogen auf das Gewicht des Trägers.

Zur Herstellung des beschriebenen Katalysators wird auf Niobsäure, Tantalsäure oder ein Gemisch beider eine Lösung eines Salzes von Ruthenium oder Palladium oder im Falle der Verwendung beider Metalle Lösungen der beiden Salze aufgetränkt, nach dem Auftränken getrocknet; der Katalysator wird in der dann vorliegenden Form oder nach einer Behandlung mit Wasserstoff bei 120-400°C eingesetzt.

Zur Herstellung wird der Katalysatorträger in der Form von Pillen, Kugeln oder Bruchstücken von etwa 1 - 10 mm benutzt. Das Auftränken der Edelmetallsalze geschieht in einer dem Fachmann grundsätzlich bekannten Weisen. Das Trocknen wird beispielsweise bei 100 - 140°C und vermindertem bis normalen Druck, etwa bei 1 - 1000 mbar, beispielsweise bei Wasserstrahlvakuum vorgenommen.

Die Edelmetallsalze können in Wasser oder in geeigneten organischen Lösungsmitteln gelöst werden. Bevorzugt werden sie in organischen Lösungsmitteln gelöst, wie in einfachen Alkoholen, Ketonen, Nitrilen oder cyclischen Ethern. Beispiele für solche Lösungsmittel sind Methanol, Ethanol, Aceton, Acetonitril und Dioxan. Geeignete Salze der Edelmetalle sind beispielsweise ihre Chloride, Nitrate oder Acetate.

Nach dem Imprägnieren und dem anschließenden Trocknen steht der erfindungsgemäß einsetzbare Katalysator grundsätzlich zur Verfügung. In bevorzugter Weise wird er jedoch vor seinem Einsatz durch Behandlung mit Wasserstoff bei erhöhter Temperatur aktiviert. Eine solche erhöhte Temperatur liegt im Bereich von 120-400°C, bevorzugt im Bereich von 150-340°C.

Die beschriebenen Katalysatoren können in hervorragender Weise zur Kernhydrierung von gegegebenenfalls substituiertem Anilin verwendet werden. Hierbei entsteht ein Gemisch von Cyclohexylamin und Dicyclohexylamin, welches das Substitutionsmuster des eingesetzten Anilins aufzeigt. In besonders überraschender Weise läßt sich unter Verwendung der erfindungsgemäßen Katalysatoren in Abhängigkeit von der Hydriertemperatur die Menge des dabei mit entstehenden Dicyclohexylamins gegenüber dem Monocyclohexylamin verändern, wodurch eine gezielte Herstellung von Dicyclohexylamin in größeren Mengen möglich ist.

Das erfindungsgemäße Verfahren läßt sich in einem weiten Druckbereich ausüben, der von einem Druck in der Nähe des Normaldrucks bis zu sehr hohem Druck reicht. Denkbare Ausführungsformen umfassen bespielsweise das Arbeiten in der Gasphase in der Nähe des normalen Drucks, das Arbeiten in einem Autoklaven bei hohem Druck und das Arbeiten in der Rieselphase ebenfalls unter hohem Druck.

Die Hydrierung an den beschriebenen Katalysatoren kann demnach sowohl diskontinuierlich als auch kontinuierlich erfolgen; für technische Zwecke wird in bevorzugter Weise kontinuierlich gearbeitet. In vorteilhafter Weise arbeitet man hierbei, wie bereits genannt, in der Rieselphase an einer fest angeordneten Katalysatorschüttung. Als Katalysatorbelastung wird hierbei eine Menge von 0,05 - 3 kg, bevorzugt 0,1 - 2 kg, besonders bevorzugt 0,15 - 1,5 kg, gegebenenfalls substituiertes Anilin pro Liter Katalysator und Stunde eingestellt. Eine geringe Veränderung des erzielten Anteils an Dicyclohexylamin sowie an Umsatz und Selektivität durch die sich verändernde Aktivität des Katalysators im Verlaufe längerer Reaktionsperioden können durch ein geringes Nachstellen der Reaktionstemperatur oder der anderen Parameter ausgeglichen werden. Diese Verhältnisse können anhand der Analytik des Reaktionsgemisches verfolgt werden. Als Einsatzmaterialien kommen im Sinne der folgenden Reaktionsgleichung Anilin und substituierte Aniline in Betracht, die zu den korrespondierenden Cyclohexylaminen und Dicyclohexylaminen umgesetzt werden: 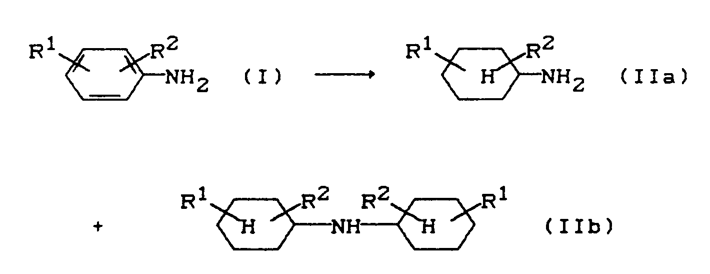
Die Reste R¹ und R² haben unabhängig voneinander die Bedeutung von Wasserstoff, C₁-C₄-Alkyl oder C₁-C₄-Alkoxy. Beispiele für die genannten Alkyl- bzw. Alkoxysubstituenten sind: Methyl, Ethyl, Propyl, Isopropyl, Butyl, Isobutyl, Methoxy, Ethoxy, Propoxy, Isopropoxy, Butoxy oder Isobutoxy. In bevorzugter Weise haben die genannten Substituenten 1-2 C-Atome, besonders bevorzugt handelt es sich um Methyl, bzw. um Methoxy. In weiterhin bevorzugter Weise hat einer der Substituenten R¹ und R² die Bedeutung Wasserstoff, während der andere Substituent Wasserstoff bzw. Alkyl bzw. Alkoxy im genannten Umfang bedeutet. In besonders bevorzugter Weise richtet sich das erfindungsgemäße Verfahren auf die Kernhydrierung von nicht substituiertem Anilin.

Als substituiertes Anilin ist erfindungsgemäß weiterhin ein am Stickstoff durch die Cyclohexylidengruppe substistuiertes Anilin zu verstehen, welches in einfacher Weise durch Kondensation von Anilin und Cyclohexanon hergestellt werden kann, Ein solches Cyclohexylidenanilin kann ebenfalls kernsubstituiert sein und entspricht dann der Formel 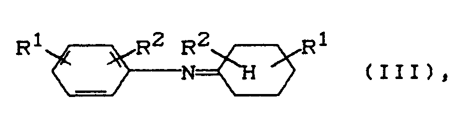
worin R¹ und R² den gegebenen Bedeutungsumfang haben, Es ist schließlich weiterhin möglich, ein solches gegebenenfalls substituiertes Cyclohexyliden-anilin auch in Form eines Gemisches des zugrundeliegenden gegebenenfalls substituierten Cyclohexanons mit dem zugrundeliegenden gegebenenfalls substituierten Anilin einzusetzen.

Cyclohexylamine und Dicylohexylamine des genannten Bedeutungsumfanges finden Verwendung zur Herstellung von Alterungsschutzmitteln für Kautschuke und Kunststoffe, als Korrosionsschutzmittel, sowie als Vorprodukt für Pflanzenschutzmittel und Textilhilfsmittel.

### Beispiel 1

75 g Niobsäure, Nb₂O₅.nH₂O, die unter Zusatz von 3,5 % Graphitpulver zu 5 mm-Tabletten verformt worden waren, wurden mit einer Lösung getränkt, die aus 1,56 g Pd-Acetat und 26,8 g Acetonitril hergestellt worden war. Die auf diese Weise mit 1 % Pd imprägnierten Niobsäure-tabletten wurden 18 Stunden bei 100°C unter Wasserstrahlvakuum getrocknet. Danach wurde die Niobsäure nochmals auf die gleiche Weise mit 1 % Pd imprägniert. 60 ml (61 g) des hergestellten Katalysators wurden in ein senkrecht angeordnetes Druckrohr (Durchmesser 14 mm, Lange 70 cm) gebracht, das mit einem Ölthermostat beheizt wurde. Das Zwischenkornvolumen wurde mit feinem Seesand (0,2 - 0,4 mm) ausgefüllt. Der Katalysator wurde zunächst 3 Stunden bei 250°C und 270 bar mit Wasserstoff aktiviert, wobei stündlich 40 l Wasserstoff am unteren Ende des Reaktionsrohres entspannt wurden. Anschließend wurde bei 270 bar und 182°C mit der Hydrierung von Anilin begonnen, wobei Anilin und Wasserstoff von oben auf den Katalysator geleitet wurden. Die Flüssigkeit rieselte über den Katalysator nach unten in einen Abscheider. Am Kopf des Abscheiders wurden 26 bis 62 Liter Wasserstoff pro Stunde entspannt. Die kontinuierlich eingespeiste Anilinmenge entsprach einer Katalysatorbelastung im Bereich von 0,23 - 0,28 g/ml Katalysator/h. Bei stationären Reaktionsbedingungen ergab sich folgende produktzusammensetzung in Abhängigkeit von den Betriebsstunden und den Reaktionstemperaturen (der Rest zu 100 % sind Nebenprodukte).

| Zeit h | Temperatur °C | DCHA*⁾ % | CHA*⁾ % |
|---|---|---|---|
| 47 | 181 | 82,0 | 17,8 |
| 119 | 171 | 84,5 | 15,3 |
| 263 | 150 | 88,5 | 11,4 |
| 360 | 139 | 91,8 | 8,0 |

| | | | |
|---|---|---|---|
| *⁾ DCHA = Dicylohexylamin; CHA = Cyclohexylamin | | | |

### Beispiel 2

100 g Niobsäure-Tabletten der gleichen Art wie in Beispiel 1 wurden mit einer Lösung getränkt, die aus 2,08 g Pd-Acetat und 25 g Dioxan hergestellt worden war. Die mit 1 % Pd imprägnierten Niobsäure-Tabletten wurden 18 Stunden bei 100°C getrocknet.

Mit 60 ml (59,7 g) des hergestellten Katalysators wurde die Hydrierung von Anilin in gleicher Weise wie in Beispiel 1 vorgenommen. Die kontinuierlich eingesetzte Anilinmenge entsprach einer Katalysatorbelastung von 0,25 g Anilin/ml Kat. x h. In Abhängigkeit von den Betriebsstunden des Katalysators sowie der Hydriertemperatur ergab sich folgende Produktzusammensetzung (der Rest zu 100 % sind Nebenprodukte):

| Zeit h | Temperatur °C | DCHA*⁾ % | CHA*⁾ % | Anilin % |
|---|---|---|---|---|
| 98 | 179 | 84,2 | 15,6 | - |
| 219 | 179 | 83,8 | 16,0 | 0,05 |
| 411 | 180 | 86,7 | 13,1 | 0,04 |
| 578 | 189 | 85,7 | 13,9 | - |
| 748 | 189 | 86,5 | 13,3 | - |
| 895 | 189 | 86,4 | 13,5 | - |
| 940 | 170 | 92,2 | 7,5 | 0,13 |

| | | | | |
|---|---|---|---|---|
| *⁾ DCHA = Dicylohexylamin; CHA = Cyclohexylamin | | | | |

### Beispiel 3

50 g Niobsäure-Tabletten (d = 5 mm) wurden mit einer Lösung getränkt, die aus 3,13 g Ru(NO₃)₂ und 25 g Methanol hergestellt worden war, Die auf diese Weise mit 2 % Ru imprägnierte Niobsäure wurde 18 Stunden bei 100°C in Wasserstrahlvakuum getrocknet. 25 ml (24 g) des so hergestellten Katalysators wurden zur Anilinhydrierung in einem 250 ml-Schüttelautoklaven verwendet, der im Inneren mit einem zentral gelagerten, fest mit dem Autoklaven verbundenen Siebkorb ausgestattet war, in den der Katalysator gefüllt wurde. Mit dieser Katalysatorfüllung wurden je 50 g Anilin bei einem Wasserstoffdruck von 260 bis 280 bar bei verschiedenen Temperaturen hydriert. Die Hydrierzeit betrug bei jedem Hydrierversuch drei Stunden. Die Reaktionsprodukte zeigten in Abhängigkeit von der Reaktionstemperatur folgende Zusammensetzung (Rest zu 100 % sind Nebenprodukte):

| Temperatur (°C) | 110 | 200 |
|---|---|---|
| Anilin | 0,4 % | 0,5 % |
| Cyclohexylamin | 90,7 % | 48,2 % |
| Dicyclohexylamin | 8,5 % | 50,0 % |

### Beispiel 4

80 g Nb₂O₅.nH₂O, die unter Zusatz von 1,9 % Graphit tablettiert (d = 5 mm) worden waren, wurden mit einer Lösung getränkt, die aus 0,83 g Pd-Acetat und 17,2 g Acetonitril hergestellt wurde. Nach einer Zwischentrocknung - 18 Stunden bei 100°C im Wasserstrahlvakuum - wurden die Niobsaure-Tabletten nochmals mit einer Losung getränkt, die aus 1,25 g Ru(NO₃)₂ und 17,2 g Methanol hergestellt wurde. Nach einer erneuten Trocknung war der Katalysator für die Hydrierung einsatzbereit. Die Niobsäure-Tabletten enthielten 0,5 % Pd und 0,5 % Ru.

25 ml (25,3 g) des so hergestellten Pd-Ru-Katalysators wurde zur Hydrierung von Anilin in einem 0,25 l-Schüttelautoklaven verwendet. Der Autoklav war im Inneren mit einem Siebkorb ausgestattet, in den der Katalysator gefüllt wurde. Mit dieser Katalysatorfüllung wurden zweimal nacheinander 50 g Anilin bei einem Wasserstoffdruck von 260-280 bar und bei verschiedenen Temperaturen hydriert. Nach den einzelnen Hydrierungen wurde der Autoklav auf Raumtemperatur abgekühlt, das Reaktionsprodukt wurde entnommen und der Autoklav wieder mit Anilin beschickt. Dabei ergab sich in Abhängigkeit von der Temperatur bei einer konstanten Hydrierzeit von 3 Stunden folgende Produktzusammensetzung (Rest zu 100 % sind Nebenprodukte):

| Temperatur (°C) | 180 | 200 |
|---|---|---|
| Anilin | 0,1 % | 0,5 % |
| Cyclohexylamin | 69,8 % | 38,5 % |
| Dicyclohexylamin | 29,4 % | 56,3 % |

### Beispiel 5

15 ml (15,5 g) des analog Beispiel 1 hergestellten Katalysators wurden zur Hydrierung von Anilin in der Gasphase eingesetzt. Der Katalysator wurde zunächst eine Stunde in einem Wasserstoffstrom von 20 l/h bei 190°C aktiviert. Durch den aktivierten Katalysator, der sich in einem senkrecht angeordnetem 17 mm weiten Reaktionsrohr befand, wurden pro Stunde 1,54 g Anilin zusammen mit 20 l Wasserstoff geleitet. Das entstandene Reaktionsprodukt wurde kondensiert und nach verschiedenen Zeitabständen analysiert. Dabei wurden in Abhängigkeit von den Betriebsstunden des Katalysators folgende Zusammensetzung des Reaktionsproduktes gefunden (Rest zu 100 % sind Nebenprodukte):

| Versuchsdauer | 116 h | 238 h | 303 h | 438 h |
|---|---|---|---|---|
| Cyclohexylamin | 22,5 | 22,1 | 18,8 | 18,5 |
| Dicyclohexylamin | 74,2 | 74,6 | 77,9 | 77,9 |
| N-Cyclohexylanilin | 1,2 | 1,2 | 1,2 | 1,4 |
| Anilin | 0,4 | 0,6 | 1,2 | 0,7 |

### Beispiel 6

25 ml (24,8 g) des nach Beispiel 2 hergestellten Katalysators wurden zur Hydrierung von Cyclohexylidenanilin in einem 250 ml-Schüttelautoklaven verwendet, der im Inneren mit einem zentral gelagerten, fest mit dem Autoklaven verbundenen Siebkorb ausgestattet war, in den der Katalysator gefüllt wurde. Mit dieser Katalysatorfüllung wurden je 50 g Cyclohexylidenanilin unter einem Wasserstoffdruck von 280 bar bei verschiedenen Temperaturen hydriert. Im Einsatzprodukt waren etwa 95 % Cyclohexylidenanilin enthalten. Die Hydrierzeit betrug bei allen Versuchen dieser Serie 3 Stunden. Die Hydrierprodukte zeigten folgende Zusammensetzung (Rest zu 100 % sind Nebenprodukte):

| Temperatur (°C) | 200 | 100 |
|---|---|---|
| Dicyclohexylamin % | 89,1 | 89,9 |
| Cyclohexylamin % | 0,9 | 1,25 |
| N-Cyclohexylanilin | - | 3,5 |

### Beispiel 7

Unter Weiterbenutzung der in Beispiel 1 beschriebenen Apparatur samt Katalysatorfüllung (2 % Pd auf Nb₂O₅·nH₂O) wurde statt Anilin ein Gemisch von Cyclohexanon und Anilin im Molverhältnis 0,9:1 eingesetzt. Die kontinuierlich eingespeiste Eduktmenge entsprach einer Katalysatorbelastung von 0,26 g Gemisch/ml Katalysator/h. Am Kopf des Abscheiders wurden etwa 50 Liter Wasserstoff pro Stunde entspannt. Bei stationären Reaktionsbedingungen ergab sich folgende Produktzusammensetzung in Abhängigkeit von der Reaktionstemperatur (der Rest zu 100 % sind Nebenprodukte).

| Temperatur | 180°C | 141°C |
|---|---|---|
| Laufzeit | (ø aus) 47 h | (ø aus) 24 h |
| Dicyclohexylamin | 94,2 | 92,7 |
| Cyclohexylamin | 3,7 | 2,8 |
| N-Cyclohexylanilin | - | 2,8 |
| Cyclohexanol | 1,9 | 1,7 |

### Beispiel 8

400 g kugelförmiges γ-Al₂O₃ mit einem Durchmesser von 2-5 mm und einer spezifischen Oberfläche von 350 m²/g wurden mit einer Lösung von 23,3 g NbCl₅ in 120 g 37 %iger Salzsäure getränkt und dann bei 120°C getrocknet. Dann wurde der Katalysatorträger mit 410 g einer 16,9 Gew.-%igen wäßrigen Ammoniaklösung getränkt und anschließend mit Wasser chloridfrei gewaschen und wieder getrocknet. 150 g des so hergestellten Katalysatorträgers wurden mit einer Lösung getränkt, die aus 3,13 g Pd-Acetat und 40 g Acetonitril hergestellt worden war. Nach erneutem Trocknen bei 120°C war der Katalysator einsatzbereit. Mit 60 ml (53 g) des so hergestellten Katalysators wurde die Anilinhydrierung durchgeführt. Hierzu wurde der Katalysator in ein senkrecht angeordnetes Druckrohr (Innendurchmesser =14 mm, Länge ^{∼} 70 cm) gebracht. Der Katalysator wurde zunächst 3 Stunden bei 250°C und 276 Bar mit Wasserstoff aktiviert, wobei stündlich 60 l Wasserstoff hinter dem Reaktionsrohr entspannt wurden.

Danach wurde die Temperatur auf etwa 200°C gesenkt und bei 275 bar Anilin und Wasserstoff von oben auf den Katalysator geleitet. Die Flüssigkeit rieselte über den Katalystor nach unten in einen Abscheider. Am Kopf des Abscheiders wurden stündlich 60 bis 100 l/h Wasserstoff entspannt.

Der Anilindurchsatz entsprach einer Katalysatorbelastung von 0,2 bis 0,3 g Anilin/ml Kat. x h und wurde in diesem Bereich gehalten.

Das Hydrierprodukt wurde in regelmäßigen Zeitabständen aus dem Abscheider entnommen und analysiert. Dabei ergab sich folgende Produktzusammensetzung in Abhängigkeit von der Laufzeit bei einer Reaktionstemperatur von 200°C (der Rest zu 100 % sind Nebenprodukte):

| Laufzeit (h) | Anilin (%) | DHA* (%) | CHA* (%) |
|---|---|---|---|
| 89 | 0,2 | 76,1 | 22,9 |
| 137 | - | 79,2 | 20,5 |
| 212 | - | 78,2 | 21,4 |
| 255 | - | 80,6 | 18,7 |
| 326 | 0,1 | 74,3 | 24,6 |
| 374 | - | 77,1 | 22,6 |

| | | | |
|---|---|---|---|
| *) DHA = Dicyclohexylamin; CHA = Cyclohexylamin | | | |

### Beispiel 9

400 g des in Beispiel 8 verwendeten γ-Al₂O₃-Granulats wurde mit einer Niobchloridlösung getränkt, die aus 11,64 g NbCl₅, 11,64 g NaCl und 100 g Wasser hergestellt wurde. Diese Tränkung wurde 4-mal nach jeweils einer Zwischentrocknung bei 120°C wiederholt. Auf den getrockneten Katalysatorträger ließ man 1 Stunde lang 410 g einer 8,9 %igen wäßrigen Ammoniaklösung einwirken; anschließend wurde mit Wasser chloridfrei gewaschen und erneut getrocknet.

100 g des so hergestellten Katalysatorträgers wurden mit einer Lösung getränkt, die aus 4,16 g Pd-Acetat und 31 g Methylenchlorid hergestellt worden war. Nach erneutem Trocknen wurden 60 ml (51,1 g) Katalysator zur Anilinhydrierung in gleicher Weise wie in Beispiel 8 verwendet.

Bei 200°C und 275 bar wurden in 21 Stunden 368 g Anilin gleichmäßig über den Katalysator geleitet. Am Kopf des Produktabscheiders wurden stündlich 220 l Wasserstoff entspannt. Die gaschromatographische Analyse des Reaktionsproduktes ergab 23 % Cyclohexylamin und 76,9 % Dicyclohexylamin.

### Beispiel 10

4 g TaCl₅ wurden in 6,8 g einer 37 %igen Salzsäure gelöst und anschließend mit 25 g Wasser verdünnt. Mit der so hergestellten Tantal-chlorid-Lösung wurden 100 g γ-Al₂O₃-Granulat (Durchmesser = 2-5 mm, spezifische Oberfläche = 350 m²/g) imprägniert. Nach einer Zwischentrocknung bei 100°C unter Wasserstrahlvakuum wurde der Katalyatorträger mit 25 g einer 19,3 %igen wäßrigen Ammoniaklösung getränkt und danach in fließenden Wasserstrom chloridfrei gewaschen. Nach einer weiteren Zwischentrocknung wurde der Katalysatorträger mit einer Lösung getränkt, die aus 4,16 g Pd-Acetat und 35 g Methylenchlorid hergestellt worden war. Nach einer anschließenden Trocknung war der Katalysator einsatzbereit.

60 ml (49,4 g) des so hergestellten Katalysators wurden wie in Beispiel 8 zur kontinuierlichen Anilinhydrierung eingesetzt. Unter stationären Bedingungen wurden 935 g Anilin im Verlauf von 42 Stunden bei 196°C und einem Wasserstoffdruck von 270 bar über die Katalysatorschicht geleitet. Dabei wurden am Produktabscheider stündlich 140 Liter Wasserstoff entspannt. Das sich im Produktabscheider angesammelte Reaktionsprodukt enthielt 18,9 % Cyclohexylamin und 81 % Dicyclohexylamin.

## Patentansprüche

1. Verfahren zur Herstellung eines Gemisches aus gegebenenfalls substituiertem Cyclohexylamin und gegebenenfalls substituiertem Dicyclohexylamin durch katalytische Hydrierung von gegebenenfalls substituiertem Anilin an einem Edelmetall-Trägerkatalysator, dadurch gekennzeichnet, daß man einen Ruthenium, Palladium oder ein Gemisch beider enthaltenden Katalysator auf einem Träger, der Niobsäure Nb₂O₅.nH₂O, Tantalsäure Ta₂O₅.nH₂O oder ein Gemisch beider enthält, der das(die) Edelmetall(e) in einer Gesamtmenge von 0,05 - 5 Gew.-%, bevorzugt 0,1-4 Gew.-%, besonders bevorzugt 0,1-3 Gew.-% und für den Fall eines Ru/Pd-Gemisches beide Metalle in einem Gewichtsverhältnis von 1:9 - 9:1, bevorzugt 2:8 - 8:2, besonders bevorzugt 3:7 - 7:3 enthält, wobei alle Prozentsätze auf das Gesamtgewicht des Katalysators bezogen sind, einsetzt und bei einer Temperatur im Bereich von 100 - 340°C, bevorzugt 130 - 240°C und bei einem Druck von 0,5 - 500 bar, bevorzugt 2 - 400 bar, besonders bevorzugt 100 - 400 bar, ganz besonders bevorzugt 150 - 350 bar, arbeitet.

2. Verfahren nach Anspruch 1, dadurch gekennzeichnet, daß man kontinuierlich in der Rieselphase an einer fest angeordneten Katalysatorschüttung arbeitet und eine Katalysatorbelastung von 0,05 - 3 kg, bevorzugt 0,1 - 2 kg, besonders bevorzugt 0,15 - 1,5 kg, gegebenenfalls substituiertes Anilin pro Liter Katalysator und Stunde einstellt.

3. Verfahren nach Anspruch 1, dadurch gekennzeichnet, daß man ein Anilin der Formel 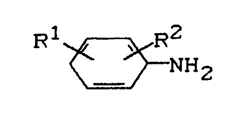 einsetzt, in der
R¹ und R² unabhängig voneinander Wasserstoff, C₁-C₄-Alkyl oder C₁-C₄-Alkoxy bedeuten.

4. Verfahren nach Anspruch 1, dadurch gekennzeichnet, daß als substituiertes Anilin das Cyclohexylidenanilin der Formel 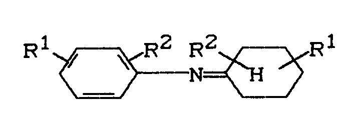 eingesetzt wird, worin
R¹ und R² unabhängig voneinander Wasserstoff, C₁-C₄-Alkyl oder C₁-C₄-Alkoxy bedeuten.

5. Verfahren nach Anspruch 4, dadurch gekennzeichnet, daß das Cyclohexyliden-anilin in Form eines Gemisches des zugrundeliegenden gegebenenfalls substituierten Cyclohexanons mit dem zugrundeliegenden gegebenenfalls substituierten Anilin eingesetzt wird.

6. Verfahren nach Anspruch 1, dadurch gekennzeichnet, daß der Träger des Katalysators Niobsäure enthält oder eine Niobsäure ist, insbesondere solche, die einen aus der natürlichen Herkunft stammenden Anteil an Tantalsäure von 0,0001 - 10 Mol %, bezogen auf die Summe der Molzahl Niob-und Tantalsäure, enthält.

7. Verfahren nach Anspruch 1, dadurch gekennzeichnet, daß der Katalysator vor seinem Einsatz mit Wasserstoff bei 120 - 400°C, bevorzugt 150 - 340°C behandelt wird.

8. Verfahren nach Anspruch 1, dadurch gekennzeichnet, daß der Katalysator einen Träger aus Al₂O₃, bevorzugt γ-Al₂O₃, mit einem Gehalt von 1-10 Gew.-% Niobsäure, Tantalsäure oder einem Gemisch beider, bezogen auf das Gewicht des Trägers, enthält.

## Claims

1. Process for preparing a mixture of optionally substituted cyclohexylamine and optionally substituted dicyclohexylamine by catalytic hydrogenation of optionally substituted aniline over a supported noble metal catalyst, characterized in that the catalyst used contains ruthenium, palladium or a mixture of the two and is on a support containing niobic acid Nb₂O₅.nH₂O, tantalic acid Ta₂O₅.nH₂O or a mixture of the two, which catalyst contains the noble metal(s) in a total amount of 0.05 - 5 % by weight, preferably 0.1 - 4 % by weight, particularly preferably 0.1 - 3 % by weight, and in the case of a Ru/Pd mixture contains the two metals in a weight ratio of 1:9 - 9:1, preferably 2:8 - 8:2, particularly preferably 3:7 - 7:3, with all percentages being based on the total weight of the catalyst, and the process is carried out at a temperature in the range of 100 - 340°C, preferably 130 - 240°C, and at a pressure of 0.5 - 500 bar, preferably 2 - 400 bar, particularly preferably 100 - 400 bar, very particularly preferably 150 - 350 bar.

2. Process according to Claim 1, characterized in that it is carried out continuously in the trickling phase over a fixed catalyst bed using a space velocity over the catalyst of 0.05 - 3 kg, preferably 0.1 - 2 kg, particularly preferably 0.15 - 1.5 kg, of optionally substituted aniline per litre of catalyst per hour.

3. Process according to Claim 1, characterized in that the aniline used is one of the formula 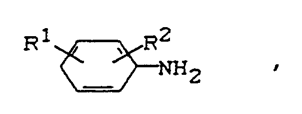 in which
R¹ and R² are, independently of one another, hydrogen, C₁-C₄-alkyl or C₁-C₄-alkoxy.

4. Process according to Claim 1, characterized in that the substituted aniline used is the cyclohexylidene-aniline of the formula 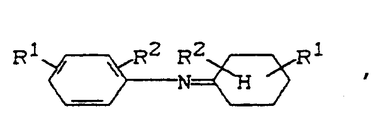 in which
R¹ and R² are, independently of one another, hydrogen, C₁-C₄-alkyl or C₁-C₄-alkoxy.

5. Process according to Claim 4, characterized in that the cyclohexylidine-aniline is used in the form of a mixture of the optionally substituted cyclohexanone on which it is based with the optionally substituted aniline on which it is based.

6. Process according to Claim 1, characterized in that the catalyst support contains niobic acid or is a niobic acid, in particular one which contains a naturally occurring proportion of tantalic acid of 0.0001 - 10 mol %, based on the sum of the number of moles of niobic and tantalic acid.

7. Process according to Claim 1, characterized in that the catalyst is, prior to use, treated with hydrogen at 120 - 400°C, preferably 150 - 340°C.

8. Process according to Claim 1, characterized in that the catalyst contains a support of Al₂O₃, preferably γ-Al₂O₃, containing 1 - 10 % by weight of niobic acid, tantalic acid or a mixture of the two, based on the weight of the support.

## Revendications

1. Procédé de fabrication d'un mélange de cyclohexylamine, éventuellement substituée, et dicyclohexylamine, éventuellement substituée, par hydrogénation catalytique d'aniline, éventuellement substituée, sur un catalyseur à base de métal noble sur un support, caractérisé en ce qu'est utilisé un catalyseur contenant du ruthénium, du palladium ou un mélange des deux sur un matériau support de catalyseur qui contient de l'acide niobique Nb₂O₅ · nH₂O de l'acide tantalique Ta₂O₅ · nH₂O ou un mélange des deux, qui contient une quantité totale du/des métal/aux noble(s) de 0,05 - 5 % en poids, de préférence 0,1 - 4 % en poids, plus particulièrement 0,1 - 3 % en poids et, dans le cas d'un mélange Ru/Pd, les deux métaux dans une proportion pondérale de 1:9 - 9:1, de préférence 2:8 - 8:2, plus particulièrement 3:7 - 7:3, tous les pourcentages étant calculés par rapport au poids total de catalyseur, et que le procédé a lieu à une température située dans la plage de 100 - 340°C, de préférence 130-- 240°C, et sous une pression de 0,5 - 500 bar, de préférence 2 - 400 bar, plus particulièrement 100 - 400 bar, la préférence absolue étant de 150 - 350 bar.

2. Procédé selon la revendication 1, caractérisé en ce que le travail s'opère en continu dans la phase de ruissellement d'un lit fixe de catalyseur, avec une charge du catalyseur de 0,05 - 3 kg, de préférence 0,1 - 2 kg, plus particulièrement 0,15 - 1,5 kg, d'aniline, éventuellement substituée, par litre de catalyseur et par heure.

3. Procédé selon la revendication 1, caractérisé en ce qu'on utilise une aniline répondant à la formule 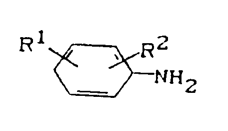 dans laquelle,
R¹ et R² désignent indépendamment l'un de l'autre l'hydrogène, un alkyle C₁ - C₄ ou un alkoxyle C₁ - C₄.

4. Procédé selon la revendication 1, caractérisé en ce qu'on utilise comme aniline substituée la cyclohexylidèneaniline répondant à la formule 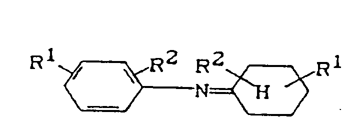 dans laquelle
R¹ et R² désignent indépendamment l'un de l'autre l'hydrogène, un alkyle C₁ - C₄ ou un alkoxyle C₁ - C₄.

5. Procédé selon la revendication 4, caractérisé en ce qu'est utilisée la cyclohexylidèneaniline sous forme d'un mélange de la cyclohexanone de base, éventuellement substituée, avec l'aniline de base, éventuellement substituée.

6. Procédé selon la revendication 1, caractérisé en ce que le matériau support du catalyseur contient de l'acide niobique ou est un acide niobique, en particulier un tel acide, qui de par son gisement d'origine possède une teneur en acide tantalique de 0,0001 - 10 % en moles, par rapport à la somme des nombres de moles d'acide niobique et d'acide tantalique.

7. Procédé selon la revendication 1, caractérisé en ce que le catalyseur, avant son utilisation, est traité à l'hydrogène à 120 - 400°C, de préférence 150 - 340°C.

8. Procédé selon la revendication 1, caractérisé en ce que le catalyseur contient un matériau support en Al₂O₃, de préférence γ-Al₂O₃, avec une teneur de 1-10 % en poids en acide niobique, acide tantalique ou un mélange des deux, par rapport au poids du matériau support.
